# EUROPEAN PATENT APPLICATION

(11) **EP 1 420 068 A1**
(43) Date of publication of application: **19.05.2004**
(21) Application number: 02746068.2
(22) Date of filing: 15.07.2002
(51) Int. Cl.: C12Q 1/68, C12N 15/00

(54) **METHOD OF ANALYZING NUCLEIC ACID SPECIFYING GENE SHOWING CHANGE IN EXPRESSION DOSE IN SCHIZOPHRENIA**

(30) Priority: 27.07.2001 JP 2001228038
(71) Applicant: Japan as represented by President of Niigata University, Niigata-shi, Niigata 950-2181 (JP)
(72) Inventor: NAWA, Hiroyuki, Niigata-shi, Niigata 951-8104 (JP); TAKAHASHI, Hitoshi, Niigata-shi, Niigata 951-8144 (JP); IRITANI, Shuji, Setagaya-ku, Tokyo 157-0063 (JP)
(74) Representative: Elsy, David
(86) International application number: PCT/JP2002/007184
(87) International publication number: WO 2003/012140

(57) **Abstract**

An object of the present invention is to contribute to objective diagnosis of schizophrenia with use of gene expression as an index. A method to analyze in a test subject whether expression of nucleic acid(s) defining gene(s) exhibiting altered expression by schizophrenia is statistically included within the range of normal subject or not is provided by the present invention. The present method comprises the step of quantifying the amount of expression of nucleic acid(s) defining gene(s) exhibiting altered expression by schizophrenia and/or protein(s) encoded by said nucleic acid(s) defining gene(s) exhibiting altered expression by schizophrenia.

## Description

### Background of the Invention

### 1. Field of the invention

The present invention relates to a method to analyze whether the amount of expression of nucleic acid(s) defining gene(s) exhibiting altered expression by schizophrenia is statistically included within the range of normal subject or not.

### 2. Prior art

Schizophrenia is a mental disorder and about 0.8% of the population suffers from schizophrenia during their youth. For it takes a long time to recover from schizophrenia, social loss caused by schizophrenia is immensely large.

Therefore, enthusiastic investigations have been made in many laboratories all over the world to develop therapies and diagnoses for schizophrenia. In particular, significant progress has been made on therapies since development of dopamine receptor antagonists such as chlorpromazine.

In contrast, diagnosis of schizophrenia is still classified based on psychological symptoms such as paranoid type, disorganized type, catatonic type and a type incapable to be classified, even in the latest US diagnostic reference "DSMIV". Therefore, diagnosis of schizophrenia finally relies upon subjective diagnosis made by the doctor in attendance, hence, diagnostic accuracy of schizophrenia has not been sufficient.

Under such circumstances, chromosomal mapping of gene responsible for schizophrenia and identification of the gene have been made enthusiastically. However, definitive reports have been not made on such gene yet.

### Summary of the Invention

The present invention was performed to solve aforementioned problems. An object of the present invention is to contribute for objective diagnosis of schizophrenia using gene(s) expression as an index, which is performed by measuring expression of nucleic acid(s).

To solve said problems, the present invention provides a method to analyze in a test subject whether expression of nucleic acid(s) defining gene(s), exhibiting altered expression by schizophrenia, is statistically included within the range of normal subject or not. The present method comprises the step of measuring expression of said nucleic acid(s) defining gene(s) exhibiting altered expression by schizophrenia and/or protein(s) encoded by said nucleic acid(s) defining gene(s) exhibiting altered expression by schizophrenia.

Hereafter, the present invention is explained in detail. However, these detailed description of preferred embodiments and examples do not mean any restriction or limitation of the scope of the present invention.

### Brief Description of the Drawings

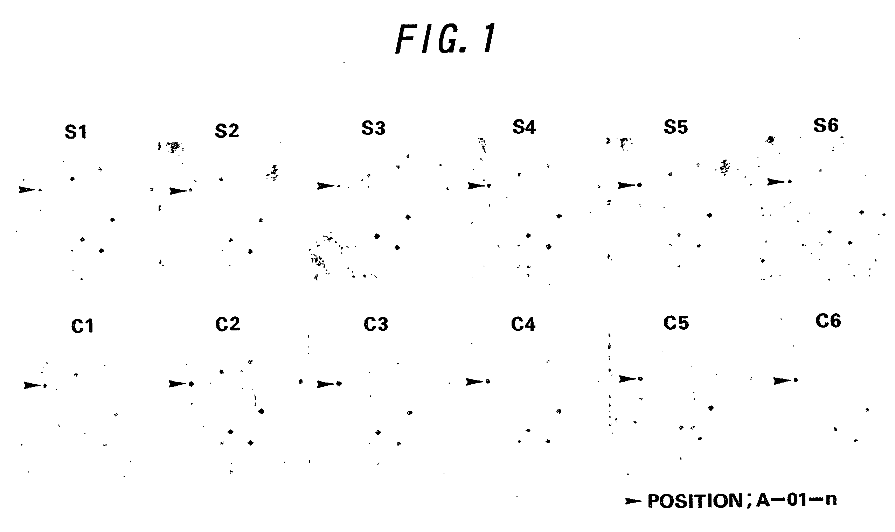

Fig. 1 is a photograph of the signals detected after hybridization using BAS5000, showing expression pattern of lysosome-associated membrane glycoprotein 2 precursor, in comparison between schizophrenic patients and individuals without mental disorder.

### Detailed Description of Preferred Embodiments

The present invention was achieved based on the knowledge obtained by the present inventors that the expression amounts of genes encoding 14 kinds of proteins listed in Table 1 described below (i.e. the expression amounts of messenger RNAs) are altered in schizophrenic patients with statistical significance. As described in detail in the following examples, the present inventors successfully identified these genes by comparing the expression amounts of about 3000 kinds of genes from autopsy brains of schizophrenic patients with those of normal individuals. Incidentally, "nucleic acid(s) defining gene(s) exhibiting altered expression by schizophrenia" in this specification means nucleic acid(s) that defines gene(s) listed in Table 1.

**Table 1-1**

| Protein encoded by nucleic acid | GenBank no. |
|---|---|
| (1) vascular endothelial growth factor precursor; VEGF | M32977 |
| (2) jun-B | M29039 |
| (3) ets domain protein elk-3 | Z36715 |
| (4) WSL protein | Y09392 |
| (5) type II cytoskeletal 8 keratin; KRT8 | M34225 |
| (6) acidic fibroblast growth factor; AFGF | X65778 |
| (7) apolipoprotein E precursor; APOE | M12529 |

**Table 1-2**

| Protein encoded by nucleic acid | GenBank no. |
|---|---|
| (8) lysosome-associated membrane glycoprotein 2 precursor; LAMP | J04183 |
| (9) beta-chimaerin | L29126 |
| (10) gamma-aminobutyric-acid receptor alpha 3 subunit precursor: GABRA3 | S62908 |
| (11) ras-related protein RAP-1A | M22995 |
| (12) gamma-glutamylcystein synthase | M90656 |
| (13) lymphocyte function-associated antigen 3 precursor; LFA3 | Y00636 |
| (14) myristoylated alanine-rich C-Kinase substrate; MARCKS | M68956 |

It was determined that the genes encoding the proteins listed in Table 1 are particularly useful as the index for diagnosis for schizophrenia, in consideration of all of the following factors:
(1) signal intensity,
(2) gene-expression alteration ratio, which is determined by selecting either of the larger one obtained from the following formula ; (i)"average expression amount in the patient group / average expression amount in the normal group" or (ii)"average expression amount in the normal group / average expression amount in the patient group" (refer to Examples),
(3) p-value obtained from test of difference in average amount of gene expression between the patient group and the normal group. Note that the term "p-value" is the probability of measuring a certain statistical value according to null hypothesis.

However, depending upon the accuracy required for the diagnosis, the index gene may be selected based upon other criteria, instead of such strict criteria (more specifically, refer to "Examples").

Specifically, the nucleic acid to be used as the index may be selected based upon the p value alone or the gene-expression alteration ratio alone.

When the index gene is selected on the basis of p value alone as the criteria, the index gene may preferably have the p value of 0.5 or less, more preferably 0.4 or less, 0.3 or less, 0.25 or less, 0.2 or less, 0.15 or less, more preferably 0.10 or less and more 0.05 or less. Further preferably, the index gene may have the p value of 0.02 or less, 0.01 or less, 0.005 or less, 0.025 or less, 0.002 or less, 0.001 or less.

When the index gene is selected on the basis of the gene-expression alteration ratio alone as the criteria, the index gene may preferably have the gene-expression alteration ratio of 1.1 or more, more preferably 1.2 or more, more preferably 1.25 or more, more preferably 1.3 or more, more preferably 1.4 or more, more preferably 1.5 or more, more preferably 1.6 or more, more preferably 1.7 or more, more preferably 1.75 or more, more preferably 1.8 or more, more preferably 1.9 or more, more preferably 2.0 or more. Further preferably the index gene may have the gene-expression alteration ratio of 2.1 or more, 2.2 or more, 2.25 or more, 2.5 or more, 3 or more, 4 or more, 5 or more, 6 or more 7 or more, 7.5 or more, not 8 or more, 9 or more, 10 or more, 15 or more, 20 or more, 25 or more, 30 or more, 40 or more, 50 or more, 60 or more, 70 or more, 75 or more.

For example, depending upon the accuracy required for the diagnosis, the expression of genes listed in the following Table 2 may be used as the index instead of or together with the genes listed in Table 1.

**Table 2**

| Protein encoded by nucleic acid | GenBank no. |
|---|---|
| (1) adenylate kinase 3; AK3 | X60673 |
| (2) interleukin-14 precursor; IL-14 | L15344 |
| (3) glial cell line-derived neurotropic factor; GDNF | L19063 |
| (4) sodium-dependent dopamine transporter | M95167 |
| (5) ferrochelatase precursor | D00726 |
| (6) LIM AND SH3 DOMAIN PROTEIN LASP-1 | X82456 |
| (7) laminin alpha-1 subunit precursor; LAMA-1 | X58531 |
| (8) golgi SNARE | AF007548 |
| (9) B-lymphocyte CD20 antigen | X12530 |
| (10) frizzled-related protein 2 | AF026692 |
| (11) placental thrombin inhibitor | S69272 |

Moreover, the method of the present invention can be utilized for the purpose to diagnose objectively whether a test subject suffers from schizophrenia or not, using the expression of the gene or fragment thereof and/or the protein encoded by the gene or fragment thereof satisfying the aforementioned criteria.

At first, to achieve the method of the present invention, a sample containing nucleic acid or protein may be obtained from a test subject to be diagnosed for schizophrenia.

According to this specification, the term "schizophrenia" includes any type of schizophrenia such as paranoid schizophrenia, disorganized schizophrenia, catatonic schizophrenia, and a type of schizophrenia incapable to be classified.

According to this specification, the term "test subject" means a human being, and particularly, the test subject may preferably be a patient which is the test subject to be diagnosed by the method of the present invention.

According to this specification, the term "test animal subject" means non-human animals, and particularly, the test subject may preferably be experimental animal such as mouse, rat, guinea pig, dog, rabbit, monkey and chimpanzee.

According to the method of the present invention, at least one protein and/or nucleic acid selected from the group consisting of the proteins listed in Table 1 and Table 2 described above, more preferably those listed in Table 1, or fragments thereof, and/or the nucleic acids encoding these proteins or fragments thereof, or fragments of said nucleic acids can be quantified.

The "nucleic acid(s) defining gene(s) encoding the protein(s) listed in Table 1 and nucleic acid(s) complementary to the nucleic acid(s)" typically means mRNA and cDNA of these proteins. Moreover, any polynucleotides, such as regulatory sequences and a polyadenyl sequences, may be included in the terminal ends of the translation region and/or inside of these mRNA or cDNA. In the case where the proteins listed in Table 1 are encoded by plural allelic genes, all of the allelic genes, their transcriptional products and cDNAs may be included in the "nucleic acid(s) defining gene(s) encoding the protein(s) listed in table 1 and the complementary nucleic acid(s) thereof".

The "fragment" of a nucleic acid means a polynucleotide including either entirely or a part of the nucleic acid defining the gene encoding the protein. Typically, it may be a restriction fragment of mRNA or cDNA encoding the protein listed in Table 1.

To quantify the expression of the index gene, at first, "sample containing nucleic-acid or protein" may be obtained from a test subject. Nucleic acid and protein widely distribute throughout a living body. Then, as long as they are derived from the same gene, they are placed under the same control. Therefore, any sample of various origins other than brain, including tissues, cells and body fluids obtained from the test subject, may be used as the "sample containing nucleic-acid or protein". Preferably, the sample may include biopsy brain, autopsy brain, cerebrospinal fluid and blood.

Particularly preferable samples may include biopsy samples obtained from origins or projection sites of dopaminergic neuron of the central nervous system. More specifically, preferable samples may include a biopsy sample obtained from caudate nucleus, putamen and so on.

The term "nucleic acid" used in this specification may include any polynucleotide consisting of simple nucleotides and/or modified nucleotides such as cDNA, mRNA, total RNA and hnRNA. The term "modified nucleotides" may include phosphoric esters such as inosine, acetylcytidine, methylcytidine, methyladenosine and methyl guanosine, as well as other postnatal nucleotides which may be produced by the effect of ultraviolet rays or chemical substances.

In general, to achieve quantification of nucleic acids, a sample may be obtained from a test subject, succeeded by procedure to extract nucleic acid form the sample. Extraction of the nucleic acid from a living body may be achieved by any extraction method such as phenol extraction and ethanol precipitation. To achieve extraction of mRNA, the sample may be passed through an oligo-dT column.

In the case where the amount of the nucleic acid is not large, the nucleic acid may be amplified, if necessary. The nucleic acid may be amplified by polymerase chain reaction (hereinafter, simply referred to as "PCR"), for example, by reverse transcriptase PCR (RT-PCR). Furthermore, as described in the following description, the amplification may be performed as a quantitative operation or the quantitative operation may be combined with other operations.

After the extraction procedure and/or the amplification procedure (if necessary) may be achieved, at least one nucleic acid or fragment thereof selected from the group consisting of nucleic acids defining genes encoding proteins listed in Table1 or 2, may be quantified.

The nucleic acid may be quantified by any known method, such as quantitative PCR, Southern blotting, Northern blotting, RNase protection mapping, or a combination of such methods.

The internal nucleotides of the amplified products may be labeled in the quantitative PCR, typically by using radio-labeled nucleotides (e.g., ³²P). Alternatively, the amplified product may be endo-labeled by using radio-labeled primers. Free radio-labeled nucleotides or radio-labeled primers may be separated from the labeled amplified products, by using some known methods including gel filtration, alcohol precipitation, trichloroacetic acid precipitaion and physical absorption using a glass filter. Thereafter, procedures such as electrophoresis and hybridization may be performed (or may not be performed) and the amplified products may be quantified by using liquid scintillation, autoradiography, and imaging plate Bio-Imaging Analyzer (BAS; Fuji Photo Film Co., Ltd.). Instead of such radioactive substance, a fluorescent substance or a luminescent substance may be used as a labeling substance, and the amplified product may be quantified by means of spectrofluorometer, fluoromicro plate reader or CCD camera. Furthermore, in the case where incorporation of the labeling substance into the amplified product is not performed during the PCR operation, an intercalate fluorescent pigment such as ethidium bromide, SYBR Green I™, PicoGreen™ (manufactured and sold by Molecular Probes) may be used to detect the amplified product.

In the case where the quantitative PCR may not be performed, the sample containing nucleic acid may be subjected to electrophoresis, and then analyzed by Southern blotting or Northern blotting, thereafter quantification may be achieved by using a probe labeled with a detectable marker.

In the case where many kinds of nucleic acids are to be quantified simultaneously, DNA chip or DNA microarray may be used together with or instead of the aforementioned techniques.

Instead of quantification of the nucleic acid or together with quantification of the nucleic acid, the amount of gene expression may be indirectly determined by quantifying the amount of protein produced from mRNA (gene). When schizophrenia is diagnosed according to the method of the present invention, in many cases, the indirect method of quantifying protein(s) encoded by nucleic acid(s) may be more useful than the direct method of quantifying nucleic acid(s).

For the method of protein extraction from tissues and for the method of protein quantification, any methods known in this field may be used. Examples of methods for protein quantification may include Western blotting method and enzyme-linked immunosorbent assay method such as solid-phase enzyme-linked immunosorbent assay, immunocytochemistry, and immunohistochemistry.

Meanwhile, only summary of the conventional procedures are schematically exemplified in this specification, therefore, modified or alternative methods of the aforementioned methods can be also utilized.

Extraction, amplification, isolation, and quantification of the nucleic acid can be performed automatically by using an automatic operation device currently on the market, in which an electrophoresis device and a PCR device and the like are combined, therefore, utilization of such device may be preferred. By using such an automatic machine, diagnosis of schizophrenia can be achieved in the same manner as routine clinical tests.

After quantification of a prescribed nucleic acid and/or protein, whether a test subject suffers from schizophrenia or not may be determined, using the quantified value as the index.

In the case diagnosis is made by using quantitative value of a singular nucleic acid and/or protein as the index, the threshold value may be determined appropriately with reference to a normal value. Then, if the quantified value is higher or lower than the threshold value, it is highly possible that the test subject suffers from schizophrenia. For example, in the case the quantified value increases in schizophrenic patients, if the quantified value is higher than the predetermined threshold, the test subject can be diagnosed to suffer from schizophrenia at high probability.

The threshold value may be selected depending upon the accurately of the diagnosis required, as shown below.

When distribution of gene expression amount is elucidated on both of the non-schizophrenic group (hereinafter, referred to as normal group) and the schizophrenic group (hereinafter, simply referred to as patient group) groups, the threshold may be selected such the manner that an individual (from which the nucleic acids or protein to be determined has been obtained) belongs to the normal group with probability of 10%, 5%, or 1%.

When distribution of gene-expression is elucidated only on the normal group, it can be hypothesized that an individual (from which the nucleic acids or protein to be determined has been obtained) belongs to the normal group. Then under this hypothesis, the threshold (the amount or concentration of nucleic acid or protein) may be determined so as to such quantified value can be obtained with a probability (hereinafter, referred to as p-value, typically two-sided probability, however, one-sided probability may be also utilized) of 10%, 5%, or 1%.

On the other hand, when distribution of gene-expression has been elucidated only on the patient group, analysis can be made in the using identical statistical method.

The p-value can be calculated by a statistical method such as t-test or non-parametric test.

To elucidate statistical distribution of gene-expression on the normal group and/or the patient group, it is generally required that at least 5 individuals, preferably 10 individuals, more preferably 20 individuals, further preferably 50 individuals and most preferably 100 individuals are to be measured.

If necessary, it is also possible to determine whether a test subject suffers from schizophrenia or not with higher accurately by using arbitrary statistic methods of various kinds, thus a diagnostic method using such statistic methods should be included within the scope of the present invention. In this specification, the step of "making statistical analysis whether the quantitative value is included within the range of the normal subject group or not" means a statistical process as described below in concrete.

In the case where diagnosis is made using the quantified value of a singular nucleic acid and/or protein as the index, as described in detail in the following Examples, the singular nucleic acid and/or protein may preferably satisfy following criteria; (1) the expression in the patient group is high (signal of 10 or more, refer to "Examples"), (2) the absolute gene-expression alteration ratio between both groups is 1.5 or more (refer to "Examples"), and (3) the p-value in the test of mean-values difference is 5% or less.

In the case where diagnosis is made using the quantified values of plural nucleic acids and/or proteins, an appropriate threshold should be determined on each of the nucleic acids and/or proteins. Then diagnosis can be made in the same manner when a singular nucleic acid and/or protein is used as the index, by examining whether the amount of gene expression is higher or lower than the threshold with respect to individual genes.

If one of the quantified values of nucleic acid(s) and/or protein(s) is higher or lower than the threshold in accordance with the accuracy required, it is possible to diagnose that the test subject may suffers from schizophrenia. If more than two quantified values of the nucleic acid(s) and/or protein(s) are higher or lower than the thresholds, it is possible that the test subject suffers from schizophrenia at higher possibility. When confirmed diagnosis is required, the more the number of the quantified values of nucleic acids and/or proteins is above or below compared with the threshold, the more accurately the diagnosis of schizophrenia can be made.

The diagnostic method of the present invention can be used together with the conventional subjective diagnostic method.

On the other hand, if quantified data on the amount of nucleic acid(s) and/or protein(s) can be collected from patients clearly suffering from schizophrenia (determined in some) and such data is applicable as the index for the diagnostic method of the present invention, it is possible to make confirmed diagnosis by the method of the present invention alone.

The subject of the present invention is to provide a method for objective diagnosis for schizophrenia, therefore, not to provide particular individual procedures for extraction, amplification and analysis described concretely in this specification. Hence, it should be noted that diagnostic method utilizing other than above-mentioned procedures are also include in the scope of present invention.

As described in the above, according to the method of the present invention, objective diagnosis can be made on whether a test subject suffers from schizophrenia or not, by using the amount of expression of nucleic acid (gene) and biological product (protein) derived from the nucleic acid (gene) as an index.

Therefore, the method of the present invention is further applicable as a method to evaluate usefulness of a model animal (excluding human beings) for schizophrenia, and a method to evaluate efficacy of a drug using such a model animal in a drug screening test.

The usefulness of a model animal for schizophrenia can be evaluated in the same manner as the diagnostic method. In concrete, the animal model can be diagnosed whether suffering from schizophrenia or not on the basis of the expression of prescribed gene(s). Then, if the test animal developed schizophrenia, the animal can be determined to be useful as an animal model for schizophrenia.

Examples of the "test animal subject" include mice, rats, and monkeys. Any animal can be employed as the "test animal subject" as long as the animal is not a human being.

Since diagnosis of schizophrenia has been more difficult on animals compared with human beings, this method is extremely useful for this purpose.

Furthermore, after administration of candidate substance as an anti-schizophrenia drug to such an animal model, the amount of prescribed nucleic acid(s) and/or protein(s) can be quantified as described above. If the animal recovers from schizophrenia or the schizophrenic condition of the animal is improved, it may be determined that said candidate substance is effective as an anti-schizophrenic agent. Hence, by applying the diagnostic method of the present invention, candidate substances as an anti-schizophrenia drug can be screened easily and accurately.

The "candidate substance as an anti-schizophrenia drug" may be any substance desired by the experimenter.

The diagnostic method of the present invention can be applied to a psychiatric assessment for the purpose to examine whether a subject is legally responsible or not, and to a psychiatric assessment performed for other purposes.

Now, the present invention will be further explained in detail with reference to Experimental Examples and Examples, which will not limits the scope of the present invention in any sense.

### Examples

We will explain the genes identified by the present inventors as a possible diagnostic index.

In this experiment, RNA was extracted from tissues, using corpus striatum of autopsy brains derived from dead schizophrenic patients (Sample group S) and those derived from non-schizophrenic individuals (Sample group C). Thereafter, the quality of the extracted RNAs was checked.

From RNAs determined to have high quality, six RNA samples are selected for each groups. Then, each of the RNAs (total 12 samples) were subjected to transcriptase reaction and labeled by radioactive phosphorus. The resultant product was used as a probe and reacted with three types of DNA microarrays (manufactured and sold by Clonetech), thereby the expression amounts of plural genes were simultaneously measured and patterning (molecular expression profile) of the genes were made. The three types of DNA microarrays used herein are Atras human 1.2 array, Atras human 1.2 array II and Atras human cancer 1.2 array (each array contains 1176 genes). Alteration of gene expression were evaluated and assayed on total of approximately 3000 genes using these three types of arrays.

Non-specific hybridization signals were eliminated from the used DNA array by washing under high temperature (65°C) and low concentration (0.3×SSC) over one hour. Then, radio-signals corresponding to individual gene spots were measured and quantified by BAS5000 image analyzer (Fuji Photo Film Co., Ltd.). In order to calibrate variation of signal intensities among DNA microarray sheets caused by experimental error, the sum value of all gene expression signals was calculated. Then the signal intensities were standardized by assuming that the sum value of all gene expression signals on the arrays was constant (total 30000), even if the array and the sample RNA differs (in general, referred to global normalization).

The signals corresponding to individual gene spots were measured and quantified by BAS5000 image analyzer (Fuji Photo Film Co., Ltd.). In order to calibrate variation of signal intensities among DNA microarray sheets which correspond to individual RNA samples, the signal intensities were standardized by assuming that the sum value of all gene expression signals on the sheets was constant, even if the sheet and the sample RNA differs.

To identify a gene exhibiting common quantitative alteration among plural schizophrenic patients, data was analyzed on the expression signals obtained from the schizophrenic patients (Sample group S; N=6) and those obtained from non-schizophrenic patients (Sample group C; N=6). Moreover, the statistical analysis was performed on signal intensity data, by using significant difference test according to the student t-test.

In the case of genes exhibiting average signal intensity larger than 10 on the schizophrenic group, if a gene exhibits an expression alteration ratio of 1.5 or more and a p value is 0.05 or less, otherwise, in the case of genes exhibiting moderate average signal intensity less than 10, if a gene exhibits an expression alteration ratio of 2.0 or more and a p value is 0.01 or less, such gene was determined as being altered significantly due to schizophrenia in this experimental example. Here, the expression alteration ratio means the larger one selected from "average expression amount in the S group/average expression amount in the C group" and "average expression amount in the C group/average expression amount in the S group". Table 1 described above is a list of genes selected on the basis of the former criteria and Table 2 described above is a list of genes selected on the basis of the latter genes. Hence, the genes listed in Table 1 selected on the basis of the aforementioned criteria are particularly useful as an index for diagnosis of schizophrenia. Moreover, the genes listed in Table 2 are also useful as an index for diagnosis of schizophrenia. Table 3 shows the detailed statistical data on the gene-expression alteration ratio, the p-value and etc. on the genes listed in Table 1. Moreover, Table 4 shows the detailed statistical data on the gene-expression alteration ratio, the p-value and etc. on the genes listed in Table 2.

**Table 3**

| Schizophrenia | | Control | | Gene Name | alteration rate | p-value (t-test) |
|---|---|---|---|---|---|---|
| Average | SD | Average | SD | | | |
| 27.10 | 11.15 | 9.840 | 8.653 | vascular endothelial growth factor precursor; VEGF | 2.754 | 0.0135 |
| 10.53 | 6.147 | 24.83 | 10.03 | jun-B | 2.359 | 0.0138 |
| 10.40 | 3.479 | 4.529 | 4.252 | ets domain protein elk-3 | 2.295 | 0.0258 |
| 16.36 | 93674 | 5.253 | 4.389 | WSL protein | 3.114 | 0.0284 |
| 17.91 | 7.260 | 8.739 | 5.368 | type II cytoskeletal 8 keratin; KRT8 | 2.049 | 0.0322 |
| 25.94 | 8.915 | 14.60 | 7.666 | acidic fibroblast growth factor; AFGF | 1.776 | 0.0399 |
| 119.5 | 33.77 | 185.1 | 43.54 | apolipoprotein E precursor; APOE | 1.549 | 0.0154 |
| 152.4 | 41.84 | 259.7 | 50.42 | lysosome-associated membrane glycoprotein 2 precursor; LAMP | 1.703 | 0.0025 |
| 13.27 | 5.042 | 25.12 | 6.770 | beta-chimaerin | 1.893 | 0.0060 |
| 10.56 | 5.203 | 3.651 | 3.651 | gamma-aminobutyric-acid receptor alpha 3 subunit precursor: GABRA3 | 3.635 | 0.0145 |
| 20.31 | 4.377 | 38.25 | 16.48 | ras-related protein RAP-1A | 1.884 | 0.0275 |
| 56.90 | 9.207 | 86.14 | 27.27 | gamma-glutamylcystein syntase | 1.514 | 0.0321 |
| 20.31 | 10.63 | 32.12 | 7.229 | lymphocyte function-associeted antigen 3 precursor; LFA3 | 1.581 | 0.0481 |
| 10.16 | 7.187 | 18.67 | 5.959 | myristolated alanin-ricg C-kinase substatrate; MARCKS | 1.837 | 0.0498 |

**Table 4**

| Schizophrenia | | Control | | Gene Name | alteration rate | p-value (t-test) |
|---|---|---|---|---|---|---|
| Average | SD | Average | SD | | | |
| 6.752 | 3.446 | 14.28 | 2.902 | adenylate kinase 3; AK3 | 2.115 | 0.0022 |
| 1.123 | 0.997 | 4.718 | 2.314 | interleukin-14 precursor; IL-14 | 4.202 | 0.0058 |
| 1.287 | 1.622 | 7.919 | 4.926 | glial cell line-derived neurotropic factor; GDNF | 6.153 | 0.0107 |
| 2.813 | 1.485 | 0.133 | 0.207 | sodium-dependent dopamine transporter | 21.19 | 0.0014 |
| 0.703 | 0.790 | 2.587 | 1.219 | ferrochelatase precursor | 3.680 | 0.0099 |
| 2.383 | 2.048 | 11.31 | 5.026 | LIM AND SH3 DOMAIN PROTEIN LASP-1 | 4.745 | 0.0024 |
| 2.323 | 3.546 | 12.35 | 6.357 | laminin alpha-1 subunit precursor; LAMA1 | 5.316 | 0.0071 |
| 6.454 | 5.244 | 18.32 | 7.133 | Golgi SNARE | 2.838 | 0.0083 |
| 0.237 | 0.432 | 10.96 | 8.007 | B-lymphohyte CD20 antigen | 46.20 | 0.0084 |
| 4.488 | 3.387 | 11.79 | 4.337 | frizzled-related protein 2 | 2.626 | 0.0087 |
| 6.825 | 6.610 | 15.80 | 2.065 | plcental thrombin inhibitor | 2.315 | 0.0099 |

For example, in the following analysis, we will explain quantification of mRNA, data processing thereof, and the possibility of diagnostic, on lysosome-associated membrane glycoprotein 2 precursor (GenBank#J04183) mRNA, which exists on the position of A-01-n of Atras human 1.2 array II and exhibits significant alteration on gene expression due to schizophrenia.
(1) To identify genes exhibiting quantitative alteration in common among plural schizophrenic patients, analysis was performed on the expression signal data obtained from the schizophrenic patients (Sample group S; N=6) and those obtained from non-schizophrenic individuals (Sample group C; N=6), according to the following example. The RNA signals of all genes existing on the DNA array containing this gene signal were measured and quantified by BAS 5000 image analyzer. The data of the expression signals obtained from the hybridization was shown in Fig. 1. As a result, the measured signal intensities of lysosome-associated membrane glycoprotein 2 precursor mRNA in respective samples was as follows.
   Schizophrenic patients (Sample group S; N=6); S1=46, S2=36, S3=40, S4=68, S5=90, S6= 58
   Non-schizophrenic individuals (Sample group C; N=6); C1=67, C2=118, C3=131, C4=97, C5=97, C6=77.
(2) According to global normalization, the signal intensities between the arrays were standardized by assuming that the sum of all gene expression signals was constant (total 30000) even if the array and the sample differs. As a result, the calibrated signal intensities of lysosome-associated membrane glycoprotein 2 precursor mRNA in respective samples was as follows.
   Schizophrenic patients (Sample group S); S1=117, S2=116, S3=94, S4=114, S5=197, S6= 195
   Non-schizophrenic individuals (Sample group C; N=6); C1=312, C2=222, C3=328, C4=233, C5=202, C6=261
(3) Using test of difference according to the student t-test, the signal data intensities were analyzed on six examples of schizophrenic patients (group S1 to S6) and on six examples of non-schizophrenic individuals (group C1 to C6).
   As a result, the average value and the standard deviation were as follows. Schizophrenic patients (sample group S); 152+/-42
   Non-schizophrenic individuals (sample group C); 260+/-50
   According to student t-test, it was revealed that average values of these two groups differ (P<0.002). Actually, in the present example, signals with signal intensities of more than 200 were all derived from schizophrenic patients and signal intensities of less than 200 were all derived from non-schizophrenic patients. Therefore, it was revealed that such criteria could be applied for determination of schizophrenia.
(4) Actually, when the standardized signal intensity of lysosome-associated membrane glycoprotein 2 precursor mRNA of unknown subject, which is obtained from DNA array analysis described above, is 152+42x1.64=221 or more, the test subject can be determined to be "normal" at the statistical significance of 95% or more, in comparison with the distribution of the schizophrenic patients (Sample group S), assuming that the schizophrenic patients are in accordance with normal distribution. Moreover, the standardized signal intensity is 260-50×1.64=178 or less, the test subject can be determined to be "schizophrenia" at the statistical significance of 95% or more, in comparison with the distribution of the non-schizophrenic individuals (Sample group C) assuming that the non-schizophrenic individuals are in accordance with normal distribution. When the signal intensity ranges from 178 to 221, the test subject could be determined to be pseudo-positive.

As described above, the genes listed in Table 1 selected according to the same criteria are particularly useful as an index for diagnosis of schizophrenia. Diagnostic reliability for schizophrenia can be improved by testing the expression amount of plural genes obtained here.

According to the method of the present invention, diagnosis can be made objectively on whether a test subject suffers from schizophrenia or not. This method enables diagnosis with high accuracy, compared with the conventional subjective diagnostic method.

## Claims

1. A method for analyzing in a test subject whether expression of nucleic acid(s) defining gene(s) exhibiting altered expression by schizophrenia is statistically included within the range of normal subject or not, the method comprising the steps of;
measuring expression of said nucleic acid(s) (containing its fragment and a nucleic acid complementary to the nucleic acid) defining gene(s) exhibiting altered expression by schizophrenia and/or protein(s) (containing its fragment) encoded by said nucleic acid(s) (containing its fragment and a nucleic acid complementary to the nucleic acid) defining gene(s) exhibiting altered expression by schizophrenia, to obtain the quantitative value of the expression in the test subject, and
making statistical analysis whether said quantitative value is included within the range of normal subject group or not,
wherein said nucleic acid(s) defining gene(s) exhibiting altered expression by schizophrenia is included in nucleic acid(s) encoding protein(s) selected from the group of proteins as described below with GenBank No described in brackets.
(1) vascular endothelial growth factor precursor (GenBank No.M32977)
(2) jun-B (GenBank No.M29039)
(3) ets domain protein elk-3 (GenBank No.236715)
(4) WSL protein (GenBank No.Y09392)
(5) type II cytoskeletal 8 keratin (GenBank No.M34225)
(6) acidic fibroblast growth factor (GenBank No.X65778)
(7) apolipoprotein E precursor (GenBank No.M12529)
(8) lysosome-associated membrane glycoprotein 2 precursor (GenBank No.J04283)
(9) β-chimaerin (GenBank No.L29126)
(10) gamma-aminobutyric-acid receptor alpha 3 subunit precursor (GenBank No.562908)
(11) ras-related protein (GenBank No.M22995)
(12) gamma-glutamylcystein synthetase (GenBank No.M90656)
(13) lymphocyte function-associated antigen 3 precursor (GenBank No.Y00636)
(14) myristoylated alanine-rich C-Kinase substrate (GenBank No.M68956)

2. A method for diagnosing whether a test subject suffers form schizophrenia or not, the method comprising the steps of;
obtaining a sample containing nucleic acid and/or protein from said subject,
measuring content of at least one of said nucleic acid(s) (containing its fragment and a nucleic acid complementary to the nucleic acid) defining gene(s) exhibiting altered expression by schizophrenia and/or at least one of protein(s) (containing its fragment) encoded by said nucleic acid(s) (containing its fragment and a nucleic acid complementary to the nucleic acid) defining gene(s) exhibiting altered expression by schizophrenia in said sample, and
diagnosing whether said test subject suffers from schizophrenia or not using index of quantitative value of said nucleic acid(s) defining gene(s) exhibiting altered expression by schizophrenia and/or at least one of protein(s) encoded by said nucleic acid(s) defining gene(s) exhibiting altered expression by schizophrenia,
wherein said nucleic acid(s) defining gene(s) exhibiting altered expression by schizophrenia is included in nucleic acid(s) encoding protein(s) selected from the group of proteins as described below with GenBank No described in brackets.
(1) vascular endothelial growth factor precursor (GenBank No.M32977)
(2) jun-B (GenBank No.M29039)
(3) ets domain protein elk-3 (GenBank No.236725)
(4) WSL protein (GenBank No.Y09392)
(5) type II cytoskeletal 8 keratin (GenBank No.M34225)
(6) acidic fibroblast growth factor (GenBank No.X65778)
(7) apolipoprotein E precursor (GenBank No.M12529)
(8) lysosome-associated membrane glycoprotein 2 precursor (GenBank No.J048183)
(9) β-chimaerin (GenBank No.L29126)
(10) gamma-aminobutyric-acid receptor alpha 3 subunit precursor (GenBank No.S62908)
(11) ras-related protein (GenBank No.M22995)
(12) gamma-glutamylcystein synthetase (GenBank No.M90656)
(13) lymphocyte function-associated antigen 3 precursor (GenBank No.Y00636)
(14) myristoylated alanine-rich-Kinase substrate (GenBank No.M68956)

3. A method for diagnosing whether a test animal subject is in schizophrenic condition or not, the method comprising the steps of;
obtaining a sample containing nucleic acid and/or protein from said test animal subject,
measuring content of at least one of said nucleic acid(s) (containing its fragment and a nucleic acid complementary to the nucleic acid) defining gene(s) exhibiting altered expression by schizophrenia and/or at least one of protein(s) (containing its fragment) encoded by said nucleic acid(s) (containing its fragment and a nucleic acid complementary to the nucleic acid) defining gene(s) exhibiting altered expression by schizophrenia in said sample, and
diagnosing whether said test animal subject is in schizophrenic condition using index of quantitative value of said nucleic acid(s) defining gene(s) exhibiting altered expression by schizophrenia and/or at least one of protein(s) encoded by said nucleic acid(s) defining gene(s) exhibiting altered expression by schizophrenia,
wherein said nucleic acid(s) defining gene(s) exhibiting altered expression by schizophrenia is included in nucleic acid(s) encoding protein(s) selected from the group of proteins as described below with GenBank No described in brackets.
(1) vascular endothelial growth factor precursor (GenBank No.M32977)
(2) jun-B (GenBank No.M29039)
(3) ets domain protein elk-3 (GenBank No.236715)
(4) WSL protein (GenBank No.Y09392)
(5) type II cytoskeletal 8 keratin (GenBank No.M34225)
(6) acidic fibroblast growth factor (GenBank No.X65778)
(7) apolipoprotein E precursor (GenBank No.M12529)
(8) lysosome-associated membrane glycoprotein 2 precursor (GenBank No.J04183)
(9) β-chimaerin (GenBank No.L29126)
(10) gamma-aminobutyric-acid receptor alpha 3 subunit precursor (GenBank No.562908)
(11) ras-related protein (GenBank No.M22995)
(12) gamma-glutamylcystein synthetase (GenBank No.M90656)
(13) lymphocyte function-associated antigen 3 precursor (GenBank No.Y00636)
(14) myristoylated alanine-rich C-Kinase substrate (GenBank No.M68956)

4. A method for determining whether a test animal subject is suitable for an animal model for schizophrenia, the method comprising the steps of,
diagnosing whether a test animal subject is in schizophrenic condition or not according to the method of Claim 3
determining that said test animal subject is suitable for an animal model for schizophrenia if said test animal is in schizophrenic condition.

5. A method for screening a possible candidate substance as an anti-schizophrenia drug, the method comprising the steps of,
administrating said substance to said animal model for schizophrenia,
diagnosing whether schizophrenic condition of said animal model is improved or cured or not by the method according to Claim 3,
determining that said substance is a possible candidate substance as an anti-schizophrenia drug if schizophrenic condition of said animal model is improved or cured.
